# EUROPEAN PATENT SPECIFICATION

(11) **EP 3 834 719 B1**
(45) Date of publication and mention of the grant of the patent: **22.01.2025**
(21) Application number: 20213556.2
(22) Date of filing: 11.12.2020
(51) Int. Cl.: A61B 5/06, A61B 5/00, A61B 5/287, A61B 18/14

(54) **TISSUE PROXIMITY INDICATION BASED ON A SUBSET OF ELECTRODES**
ANZEIGE DER GEWEBENÄHE BASIEREND AUF EINER UNTERGRUPPE VON ELEKTRODEN
INDICATION DE PROXIMITÉ DE TISSU SUR LA BASE D'UN SOUS-ENSEMBLE D'ÉLECTRODES

(30) Priority: 13.12.2019 US 201916713976
(43) Date of publication of application: 16.06.2021
(73) Proprietor: Biosense Webster (Israel) Ltd., Yokneam 2066717 (IL)
(72) Inventor: GLINER, Vladim, 2066717 Yokneam (IL); ALTMANN, Andres Claudio, 2066717 Yokneam (IL); AVNI, Uri, 2066717 Yokneam (IL)
(74) Representative: Carpmaels & Ransford LLP

(56) References cited:
- US-A1- 2019 183 378
- US-B1- 6 391 024

## Description

### FIELD OF INVENTION

The present invention relates to systems for improving medical procedures and mapping.

### BACKGROUND

Medical conditions such as cardiac arrhythmia (e.g., atrial fibrillation (AF)) are often diagnosed and treated via intra-body procedures. For example, electrical pulmonary vein isolation (PVI) from the left atrial (LA) body is performed using ablation for treating AF. PVI, and many other minimally invasive catheterizations, cause damage to targeted organ tissue to prevent electrical activity through the organ tissue.

Intra-body organs include tissue that can vary within different portion of the intra-body organ and that can also vary within different areas of chambers of the intra-body organ, such as different chambers of the heart. Accordingly, tissue proximity, as determined based on electronic signals provided by one or more electrodes may be based on the specific tissue properties at a given location of an intra-body organ, such as at different areas of a heart.

US 6391024 B1 relates to a method of assessing the adequacy of contact between an ablation electrode and biological tissue within a biological organ having biological fluid therein that includes the steps of positioning the ablation electrode proximal the biological tissue; positioning a reference electrode a distance from the ablation electrode; measuring the impedance between the ablation electrode and the reference electrode at a first frequency and measuring the impedance between the ablation electrode and the reference electrode at a second frequency. The percentage difference between the first-frequency impedance and the second-frequency impedance provides an indication of the state of electrode/tissue contact. In general, a percentage difference of at least approximately 10% serves as an indication of substantially complete electrode/tissue contact.

US 2019/183378 A1 relates to a system and method for measuring impedance across a plurality of electrodes and assessing proximity or contact between electrodes of a medical device and patient tissue. In one embodiment, contact is assessed between individual electrodes and cardiac tissue using bipolar electrode complex impedance measurements. Initially, baseline impedance values are established for each of the individual electrodes based on the responses of the electrodes to the applied drive signals. After establishing the baseline impedance values a series of subsequent impedance values are measured for each electrode.

### SUMMARY

The invention is defined in claim 1. Embodiments of the invention are defined in the dependent claims.

### BRIEF DESCRIPTION OF THE DRAWINGS

A more detailed understanding can be had from the following description, given by way of example in conjunction with the accompanying drawings wherein:
FIG. 1 is a diagram of an exemplary system of the present invention;
FIG. 2 is a flowchart for generating a location agnostic system profile in accordance with the present invention;
FIG. 3 is an illustration of a location aware system and a location agnostic system in accordance with the present invention;
FIG. 4 is an illustration of generating a location agnostic system profile in accordance with the present invention; and
FIGS. 5A and 5B show graphs corresponding to a location agnostic system and a location aware system in accordance with the present invention.

### DETAILED DESCRIPTION

Intra-body organs, such as a heart, are often mapped, examined, and/or operated on using catheter based medical procedures. During a catheter based medical procedure, a catheter with multiple electrodes may be inserted into the intra-body organ. The multiple electrodes of the catheter may be used to, for example, map the surfaces of the intra-body organ based on proximity sensing such that a surface at a given location may be mapped if a determination is made that one or more electrodes are proximate to or in contact with the surface. The number of electrodes on the catheter may determine the resolution of the data captured by a catheter. Additionally, the number of electrodes may determine the flexibility in performing electrode-based procedures such as an ablation procedure. A higher number of electrodes may result in a higher resolution such that, for example, a larger data set may be collected based on the higher number of electrodes or a finer ablation procedure may be performed based on a higher number of electrodes.

Utilization of a higher number electrodes from one or more electrodes may depend on the ability of a system to determine if the number of electrodes are proximate to (e.g., in contact with) tissue of an intra-body organ. For example, in order to determine if electrodes of a catheter are in contact with tissue of a heart chamber, a system may need to determine if the electrodes of the catheter are in contact with the tissue of the heart chamber. A proximity determination may be made by determining that the impedance sensed at the location of a tissue is above an impedance threshold for that specific location.

The impedance threshold in a first area (e.g., a first area of the heart) may be different than the impedance threshold at a second area (e.g., a second area of the heart). The impedance thresholds may vary between different areas of an intra-body organ based on properties of the tissue corresponding to the different areas of the intra-body organ. For example, properties such as tissue thickness, tissue density, tissue type, and the like may affect the impedance thresholds to determine whether an electrode or catheter is proximate (e.g., in contact) with the tissue. Accordingly, an electrode measured impedance value X (e.g., change in impedance or percentage change in impedance) at a first location may correspond to the electrode being proximate to a tissue surface at the first location (e.g., in contact with a tissue surface) whereas the same electrode measured impedance value X at a second location may not correspond to the electrode being proximate to a tissue surface at the second location.

Accordingly, a proximity determination may be based on knowledge of the location of a catheter as well as calculation of a property (e.g., impedance) of the intra-body organ as detected by the electrodes. However, location aware systems may be limited in the number of electrode signals that such a location aware system can analyze, thereby limiting the resolution that may be made available by a catheter or group of catheters that exceed the electrode count past the limit of the location aware system. The present invention enable a location agnostic system to determine proximity using a location agnostic system contact profile (i.e., a tissue contact profile for determining proximity to tissue) that is based on proximity determinations by a location aware system that is providing a subset of signals from a subset of the electrodes.

The exemplary embodiments disclosed herein may enable the use of a resource intensive location aware system that may be limited to a certain number of electrode inputs (e.g., 22 inputs as disclosed in examples herein) in combination with a high resolution location agnostic system that is capable of analyzing a greater number of inputs (e.g., 120 inputs as disclosed in examples herein). Accordingly, one advantage of implementing the exemplary embodiments disclosed herein may be to use a cost effective high resolution location agnostic system to obtain a higher resolution of data in combination with an existing lower resolution but location aware system to correlate the high resolution data with location based attributes (e.g., location based tissue impedance thresholds).

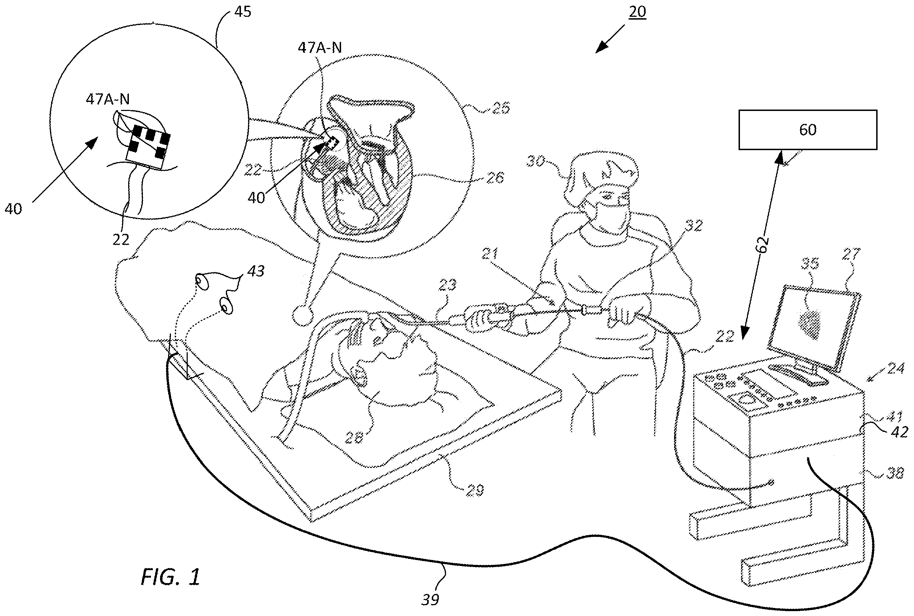

FIG. 1 is a diagram of an exemplary system 20 in which one or more exemplary features of the present invention can be implemented. System 20 may include components, such as a catheter 40, that are configured to damage tissue areas of an intra-body organ. The catheter 40 may also be further configured to obtain biometric data. Although catheter 40 is shown to be a single point catheter with multiple electrodes 47A-N, it will be understood that a catheter of any shape that includes one or more elements (e.g., electrodes) may be used to implement the embodiments disclosed herein. System 20 includes a probe 21, having shafts that may be navigated by a physician 30 into a body part, such as heart 26, of a patient 28 lying on a bed 29. According to exemplary embodiments, multiple probes may be provided, however, for purposes of conciseness, a single probe 21 is described herein but it will be understood that probe 21 may represent multiple probes. As shown in FIG. 1, physician 30 may insert shaft 22 through a sheath 23, while manipulating the distal end of the shaft 22 using a manipulator 32 near the proximal end of the catheter 40 and/or deflection from the sheath 23. As shown in an inset 25, catheter 40 may be fitted at the distal end of shaft 22. Catheter 40 may be inserted through sheath 23 in a collapsed state and may be then expanded within heart 26. Catheter 40, as set forth above, includes a plurality of electrodes 47A-N, as further disclosed herein.

According to exemplary embodiments, catheter 40 may be configured to map and/or ablate tissue areas of a cardiac chamber of heart 26. Inset 45 shows catheter 40 in an enlarged view, inside a cardiac chamber of heart 26. As shown, catheter 40 includes a plurality of electrodes 47A-N coupled onto the body of the catheter 40. According to other exemplary embodiments, multiple elements may be connected via splines that form the shape of the catheter 40. One or more other elements (not shown) may be provided and may be any elements configured to ablate or to obtain biometric data and may be electrodes, transducers, or one or more other elements.

According to exemplary embodiments disclosed herein, the electrodes, such as electrodes 47A-N, may be configured to provide energy to tissue areas of an intra-body organ such as heart 26. The energy may be thermal energy and may cause damage to the tissue area starting from the surface of the tissue area and extending into the thickness of the tissue area.

According to exemplary embodiments disclosed herein, biometric data may include one or more of LATs, electrical activity, topology, bipolar mapping, dominant frequency, impedance, or the like. The local activation time may be a point in time of a threshold activity corresponding to a local activation, calculated based on a normalized initial starting point. Electrical activity may be any applicable electrical signals that may be measured based on one or more thresholds and may be sensed and/or augmented based on signal to noise ratios and/or other filters. A topology may correspond to the physical structure of a body part or a portion of a body part and may correspond to changes in the physical structure relative to different parts of the body part or relative to different body parts. A dominant frequency may be a frequency or a range of frequency that is prevalent at a portion of a body part and may be different in different portions of the same body part. For example, the dominant frequency of a pulmonary vein of a heart may be different than the dominant frequency of the right atrium of the same heart. Impedance may be the resistance measurement at a given area of a body part.

As shown in FIG. 1, the probe 21, and catheter 40 may be connected to a console 24. Console 24 may include a processor 41, such as a general-purpose computer, with suitable front end and interface circuits 38 for transmitting and receiving signals to and from catheter, as well as for controlling the other components of system 20. In some exemplary embodiments, processor 41 may be further configured to receive biometric data, such as electrical activity, and determine if a given tissue area conducts electricity. According to an exemplary embodiment, the processor may be external to the console 24 and may be located, for example, in the catheter, in an external device, in a mobile device, in a cloud-based device, or may be a standalone processor.

As noted above, processor 41 may include a general-purpose computer, which may be programmed in software to carry out the functions described herein. The software may be downloaded to the general-purpose computer in electronic form, over a network, for example, or it may, alternatively or additionally, be provided and/or stored on non-transitory tangible media, such as magnetic, optical, or electronic memory. The example configuration shown in FIG. 1 may be modified to implement the exemplary embodiments disclosed herein. The disclosed exemplary embodiments may similarly be applied using other system components and settings. Additionally, system 20 may include additional components, such as elements for sensing electrical activity, wired or wireless connectors, processing and display devices, or the like.

According to an embodiment, a display connected to a processor (e.g., processor 41) may be located at a remote location such as a separate hospital or in separate healthcare provider networks. Additionally, the system 20 may be part of a surgical system that is configured to obtain anatomical and electrical measurements of a patient's organ, such as a heart, and performing a cardiac ablation procedure. An example of such a surgical system is the Carto^{®} system sold by Biosense Webster.

The system 20 may also, and optionally, obtain biometric data such as anatomical measurements of the patient's heart using ultrasound, computed tomography (CT), magnetic resonance imaging (MRI) or other medical imaging techniques known in the art. The system 20 may obtain electrical measurements using catheters, electrocardiograms (EKGs) or other sensors that measure electrical properties of the heart. The biometric data including anatomical and electrical measurements may then be stored in a memory 42 of the mapping system 20, as shown in FIG. 1. The biometric data may be transmitted to the processor 41 from the memory 42. Alternatively, or in addition, the biometric data may be transmitted to a server 60, which may be local or remote, using a network 62.

Network 62 may be any network or system generally known in the art such as an intranet, a local area network (LAN), a wide area network (WAN), a metropolitan area network (MAN), a direct connection or series of connections, a cellular telephone network, or any other network or medium capable of facilitating communication between the mapping system 20 and the server 60. The network 62 may be wired, wireless or a combination thereof. Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology generally known in the art. Additionally, several networks may work alone or in communication with each other to facilitate communication in the network 62.

In some instances, the server 62 may be implemented as a physical server. In other instances, server 62 may be implemented as a virtual server a public cloud computing provider (e.g., Amazon Web Services (AWS) ^{®}).

Control console 24 may be connected, by a cable 39, to body surface electrodes 43, which may include adhesive skin patches that are affixed to the patient 30. The processor, in conjunction with a current tracking module, may determine position coordinates of the catheter 40 inside the body part (e.g., heart 26) of a patient. The position coordinates may be based on impedances or electromagnetic fields measured between the body surface electrodes 43 and the electrode 48 or other electromagnetic components of the catheter 40. Additionally or alternatively, location pads may be located on the surface of bed 29 and may be separate from the bed 29.

Processor 41 may comprise real-time noise reduction circuitry typically configured as a field programmable gate array (FPGA), followed by an analog-to-digital (A/D) ECG (electrocardiograph) or EMG (electromyogram) signal conversion integrated circuit. The processor 41 may pass the signal from an A/D ECG or EMG circuit to another processor and/or can be programmed to perform one or more functions disclosed herein.

Control console 24 may also include an input/output (I/O) communications interface that enables the control console to transfer signals from, and/or transfer signals to electrodes 47A-N.

During a procedure, processor 41 may facilitate the presentation of a body part rendering 35 to physician 30 on a display 27, and store data representing the body part rendering 35 in a memory 42. Memory 42 may comprise any suitable volatile and/or non-volatile memory, such as random-access memory or a hard disk drive. In some exemplary embodiments, medical professional 30 may be able to manipulate a body part rendering 35 using one or more input devices such as a touch pad, a mouse, a keyboard, a gesture recognition apparatus, or the like. For example, an input device may be used to change the position of catheter 40 such that rendering 35 is updated. In alternative exemplary embodiments, display 27 may include a touchscreen that can be configured to accept inputs from medical professional 30, in addition to presenting a body part rendering 35.

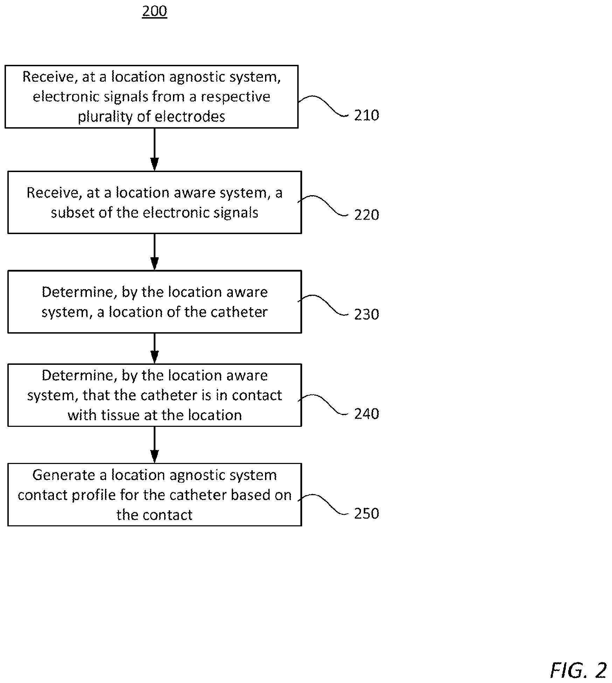

As shown in the process flow chart 200 of FIG. 2, at step 210, a plurality of electronic signals may be received from a respective plurality of electrodes. The plurality of electrodes may be attached to or part of one or more catheters. The one or more catheters may be inserted into an intra-body organ via an incision or via a natural orifice and may be directed to the intra-body organ. The plurality of electrodes may transmit electronic signals (e.g., voltage signals) to a processor (e.g., processor 41 of FIG. 1) via a wired or wireless connection. The processor may calculate impedance values based on the electronic signals provided by the plurality of electrodes and the impedance values may be applied to determine if the catheter or, more specifically, one or more of the plurality of electrodes are proximate (e.g., in contact) with the tissue of the intra-body organ, as further disclosed herein.

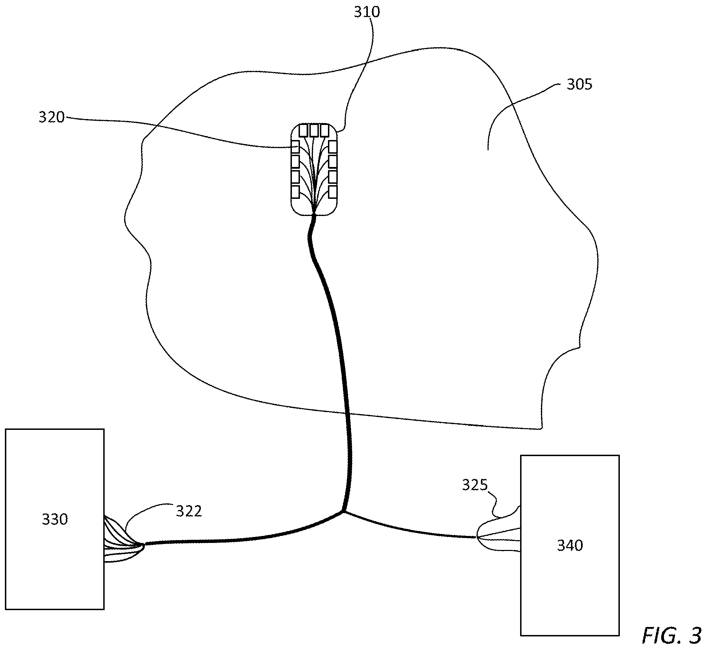

The plurality of electronic signals sensed by respective plurality of electrodes may be provided to/received by a location agnostic system. A location agnostic system may be any applicable system that is not aware of the location of the plurality of electrodes within the intrabody organ. The location agnostic system may include a processor (e.g., processor 40 of FIG. 1), a memory, and other components configured to at least determine proximity in accordance with the techniques disclosed herein. As an example, as shown in FIG. 3, the one or more catheters 310 inserted into an intra-body organ 305 that provide the respective plurality of electronic signals may have one hundred twenty (120) electrodes 320. The electronic signals 322 sensed by the one hundred twenty electrodes 320 may be provided to a location agnostic system 330 such that the location agnostic system 330 is configured to receive and process all one hundred twenty electronic signals 322 corresponding to the one hundred and twenty electrodes.

At step 220 of the process 200 of FIG. 2, a subset of the plurality of electronic signals sensed by the plurality of electrodes may be split and may be provided to a location aware system. Accordingly, the plurality of electronic signals may be provided to the location agnostic system, as described at step 210, and a subset of the plurality of electronic signals may be provided to both the location agnostic system (i.e., as described at step 210, as part of the plurality of electronic signals) and to the location aware system (i.e., as described in this step 220). Continuing the example provided herein, as shown in FIG. 3, of the one hundred twenty electronic signals 322 that are provided to the location agnostic system 330, a subset 325 of twenty-two (22) electronic signals may be split such that they are also provided to a location aware system 340.

As described herein, a location agnostic system 330 may not receive or otherwise generate location information corresponding to the one or more catheters within an intra-body organ. Accordingly, the location agnostic system 330 may not be able to determine if a catheter or, more specifically, one or more electrodes are proximate to (e.g., in contact with) tissue of the intra-body organ. Notably, the location agnostic system 330 may not be able to determine if the catheter is proximate to such tissue without the location of the catheter because such proximity determinations may require location information to determine the correct impedance thresholds such that proximity can be determined based on whether received electronic signals meet the location specific impedance thresholds for tissue at the location.

At step 230 of the process 200 of FIG. 2, a location of a catheter and, more specifically, of one or more electrodes may be determined by the location aware system. The location of the catheter may be determined based on one or more of electromagnetic transmissions, body surface electrodes, a location pad, a mapping system, or the like. For example, the location aware system may be configured to receive electromagnetic signals between a catheter and a location pad and, based on the electromagnetic signals, may determine the catheter's location. As another example, the location aware system may compare electromagnetic signals from the catheter to body surface electrode signals to determine the catheter's location relative to the body surface electrodes. Continuing the example, as shown in FIG. 3, the location aware system 340 may determine the location of the one or more catheters 310

At step 240 of the process 200 of FIG. 2, a determination that the catheter and, more specifically, one or more electrodes, is proximate to (e.g., in contact with) the tissue of the intra-body organ may be made by the location aware system. The determination that the catheter is proximate to the tissue of the intra-body organ may be based on the location of the catheter and an impedance determined based on the electrical signals sensed by the one or more electrodes. Notably, an impedance threshold may be determined based on the location of the catheter, as provided by the location aware system. The impedance threshold may be specific to the tissue at the location of the catheter such that a proximity determination can be made based on the location specific impedance threshold. The location aware system may determine that the catheter is proximate to (e.g., in contact with) the tissue of the intra-body organ based on the applicable location specific impedance threshold and the impedance value sensed by one or more of the electrodes, such that the impedance value exceeds the location specific impedance threshold to indicate proximity (e.g., contact).

At step 250 of the process 200 of FIG. 2, a location agnostic system profile for the catheter may be generated based on the proximity determination by the location aware system at step 240. Notably, when the location aware system determines that a catheter is proximate to (e.g., in contact with) tissue, at step 240, the location agnostic system may also determine impedance values based on electrical signals sensed by the one or more electrodes. The location agnostic system may generate a location agnostic system profile based on such impedance values such that the profile includes the impedance values determined by the location agnostic system while the location aware system generates a proximity determination.

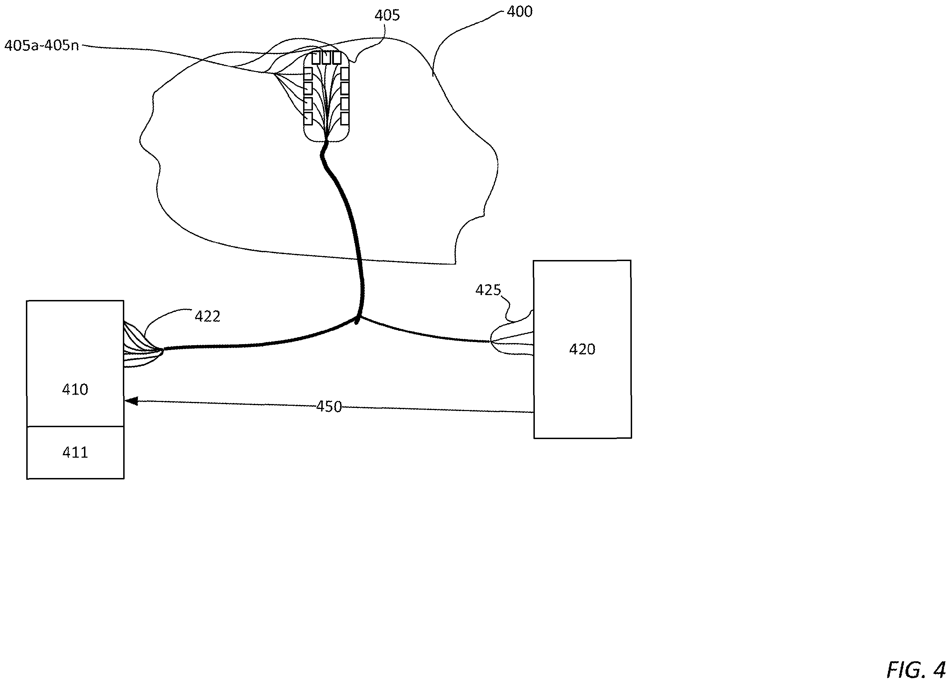

FIG. 4 shows an example illustration for generating a location agnostic system profile. As shown in FIG. 4, a catheter 405 comprising a plurality of electrodes 405a-405n may be inserted into a heart chamber 400. Electronic signals 422 from the plurality of electrodes 405a-405n may be provided to location agnostic system 410. Additionally, a subset 425 of the electronic signals from the plurality of electrodes 405a-405n may be provided to a location aware system 420.

The location aware system 420 is aware of the location of the catheter 405 and, based on the location of the catheter 405, applies an impedance threshold to determine proximity (e.g., contact) with the tissue of the heart chamber 400. The location aware system 420 determines that the subset of the electronic signals from the plurality of electrodes 405a-405n are in contact with the surface of tissue in the heart chamber 400, at a first time, based on a change in impedance values such that the change in impedance values exceeds the threshold impedance. The location aware system 420 provides a contact indication 450 to the location agnostic system 430 upon determining contact with the intra-body organ tissue. According to an implementation, the percentage of change in impedance may be applied when determining if an impedance value exceeds the threshold impedance. Upon detection of the contact, the location agnostic system 410 records the impedance values sensed by the plurality of electrodes 405a-405n at the first time. Notably, the impedance values determined by the location aware system 420 may differ from the impedance values determined by the location agnostic system 410 at the first time. However, based on the contact determination by the location aware system 420, the impedance values determined by the location agnostic system 410 for the plurality of electrodes 405a-405n are stored as the location agnostic system profile 411 such that subsequent determination of impedance values that meet the location agnostic system profile are marked as contact with the tissue at the location.

At a second time, after the first time, the location agnostic system 410 may apply the location agnostic system profile to determine if the plurality of electrodes 405a-405n are in contact with the surface of tissue of the heart chamber 400. For example, the location agnostic system profile may be applied to a set of electrical signals received by the location agnostic system 410 at the second time. Electrodes that sense electronic signals that correspond to impedance values greater than those in the location agnostic system profile may be determined to be in contact with the tissue of heart organ 400. Electronic signals that correspond to impedance values greater than those in the location agnostic system profile may be considered to meet the location agnostic system profile such that they exceed impedance thresholds as provided in the location agnostic system profile.

After the determination of the location agnostic system profile, the location aware system 420 may not be needed to determine contact with the tissue surface of the heart chamber 400 at the location. According to an exemplary embodiment of the present invention, the location aware system 420 may be disconnected at the second time such that electrical signals from the plurality of electrodes are only provided to the location agnostic system 410. Based on the process 200 of FIG. 2, a number of location agnostic system profiles may be generated and stored in memory for a number of different locations.

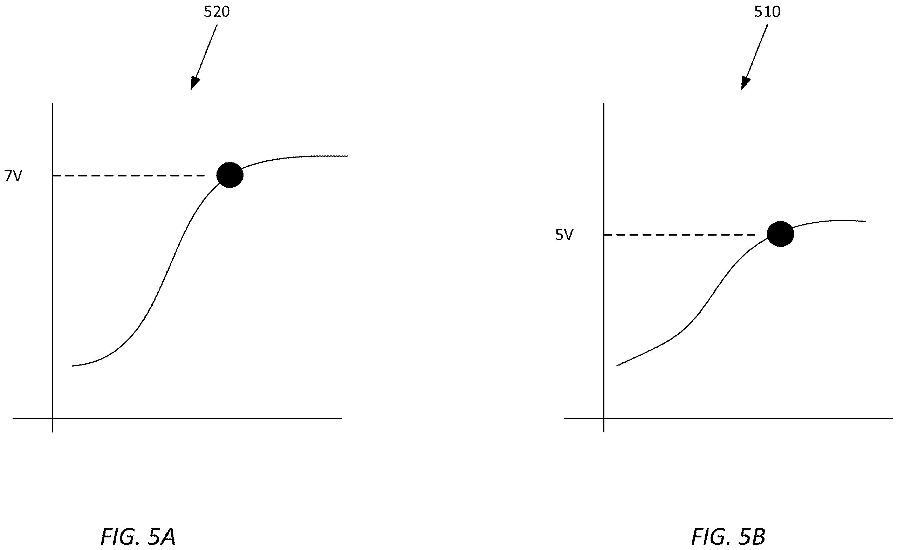

FIG. 5B shows a graph 510 corresponding to voltages detected by the location agnostic system 410 of FIG. 4 and FIG. 5A shows a graph 520 corresponding to voltages detected by the location aware system 420 of FIG. 4. Although graphs 510 and 520 show the voltage corresponding to a single electrical signal from a single electrode, it will be understood that multiple electronic signals may be used to determine a location agnostic system profile.

As shown in FIGS. 5A and 5B, the voltage difference determined by the location aware system 420 shown in graph 520 may be 7 volts when the location aware system 420 determines that the corresponding electrode is in contact with the tissue surface of the heart organ 400 of FIG. 4, based on a location specific impedance threshold. The corresponding voltage difference determined by the location agnostic system 410 may be 5 volts at the same time. Accordingly, a location agnostic system profile may be determined such that a 5 volt difference determined by the location agnostic system at the location may correspond a contact with the tissue of the heart organ 400, as determined by the location agnostic system.

Notably, a location aware system (e.g., 420 of FIG. 4) may determine that a subset of electrodes are proximate to (e.g., in contact with) tissue of an intra-body organ at a specific location. The determination may be made based on the subset of electrodes sensing an impedance value (e.g., current and voltage) that exceeds the impedance threshold for that specific location, as determined by the location aware system. A location agnostic system (e.g., 410 of FIG. 4) may also sense impedance values by the entire set of electrodes (i.e., a greater number of electrode based electrical signals than those provided to the location aware system). Based on the proximity determined by the location aware system, the location agnostic system may generate a location agnostic contact profile such that the impedance values determined by the location agnostic system, when the location aware system indicates proximity, are recorded as the impedance values that indicate contact for the location agnostic system. As noted herein, such one or more impedance values of a location agnostic contact profile may be different for the location agnostic system than those sensed by the location aware system (e.g., FIGS. 5A and 5B), even when the same signals are provided to the two different systems (e.g., FIG. 4). The differences may be due to any applicable reason such as circuitry, electricity propagation, internal components, conversion mechanisms, or the like. Accordingly, a location agnostic contact profile with at least one impedance threshold may be generated for the location agnostic system and may be used to determine proximity of the one or more catheters, by the location agnostic system, at the specific location.

Proximity (e.g., contact) indicated by the location agnostic system based on a location agnostic system profile may be used to map the surfaces of all or a part of an intra-body organ such as a heart chamber. Alternatively, or in addition, proximity indicated by the location agnostic system may be used to initiate ablation by an ablation electrode during a medical procedure.

Any of the functions and methods described herein can be implemented in a general-purpose computer, a processor, or a processor core. Suitable processors include, by way of example, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer-readable media). The results of such processing can be maskworks that are then used in a semiconductor manufacturing process to manufacture a processor which implements features of the disclosure.

Any of the functions and methods described herein can be implemented in a computer program, software, or firmware incorporated in a non-transitory computer-readable storage medium for execution by a general-purpose computer or a processor. Examples of non-transitory computer-readable storage mediums include a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs).

It should be understood that many variations are possible based on the disclosure herein. The scope of the invention is defined by the claims.

## Claims

1. A system (20) for determining a tissue contact profile for determining proximity of a catheter to tissue, the system comprising:
a catheter (310) comprising a plurality of electrodes (320) configured to sense a first plurality of electronic signals within an intra-body organ;
a location aware system (340) configured to:
receive a subset of electronic signals from the first plurality of electronic signals;
determine an impedance value based on the subset of electronic signals;
determine that the impedance value is greater than an impedance value threshold of the location;
determine a location of the catheter within the intra-body organ; and
determine that the catheter is in contact with intra-body organ tissue at the location, based on the subset of electronic signals and at least one tissue property at the location, wherein the tissue property is an impedance threshold for the location;
a location agnostic system (330) configured to:
receive the first plurality of electronic signals each from the respective plurality of electrodes of the catheter; and
generate, at a first time, the tissue contact profile for determining proximity to tissue for the catheter based on the determination, by the location aware system, that the catheter is in contact with the tissue at the location,
wherein the tissue contact profile comprises impedance values determined by the location agnostic system for the plurality of electrodes when the location aware system has determined that the catheter is in contact with the tissue at the location.

2. The system of claim 1, wherein the location agnostic system is further configured to:
receive, at a second time, a second plurality of electronic signals from the plurality of electrodes;
determine that the second plurality of electronic signals meet the tissue contact profile; and
determine that the catheter is in contact with the location based on determining that the second plurality of electronic signals meet the tissue contact profile.

3. The system of claim 2, wherein determining that the second plurality of electronic signals meet the tissue contact profile comprises determining that an impedance value corresponding to the second plurality of electronic signals is greater than an impedance value of the tissue contact profile.

4. The system of claim 1, wherein the impedance threshold for the location is based on a change in impedance.

5. The system of claim 1, wherein the impedance threshold for the location is based on a percentage of change in impedance.

6. The system of claim 1, wherein the tissue contact profile comprises one or more impedance thresholds for electrodes of the location agnostic system.

## Patentansprüche

1. System (20) zum Bestimmen eines Gewebekontaktprofils zum Bestimmen einer Nähe eines Katheters zu Gewebe, das System umfassend:
einen Katheter (310), umfassend eine Vielzahl von Elektroden (320), die konfiguriert sind, um eine erste Vielzahl von elektronischen Signalen innerhalb eines innerkörperlichen Organs zu erfassen;
ein stellenabhängiges System (340), das konfiguriert ist zum:
Empfangen einer Teilmenge von elektronischen Signalen aus der ersten Vielzahl von elektronischen Signalen;
Bestimmen eines Impedanzwerts basierend auf der Teilmenge von elektronischen Signalen;
Bestimmen, dass der Impedanzwert größer als eine Impedanzwertschwelle der Stelle ist;
Bestimmen einer Stelle des Katheters innerhalb des innerkörperlichen Organs; und
Bestimmen, dass der Katheter an der Stelle mit innerkörperlichem Organgewebe in Kontakt ist, basierend auf der Teilmenge von elektronischen Signalen und mindestens einer Gewebeeigenschaft an der Stelle, wobei die Gewebeeigenschaft eine Impedanzschwelle für die Stelle ist;
ein stellenunabhängiges System (330), das konfiguriert ist zum:
Empfangen der ersten Vielzahl von elektronischen Signalen jeweils von der jeweiligen Vielzahl von Elektroden des Katheters; und
Erzeugen, zu einem ersten Zeitpunkt, des Gewebekontaktprofils zum Bestimmen der Nähe des Katheters zu dem Gewebe basierend auf der Bestimmung, durch das stellenabhängige System, dass der Katheter an der Stelle mit dem Gewebe in Kontakt ist,
wobei das Gewebekontaktprofil Impedanzwerte umfasst, die durch das stellenunabhängige System für die Vielzahl von Elektroden bestimmt werden, wenn das stellenabhängige System bestimmt hat, dass der Katheter an der Stelle mit dem Gewebe in Kontakt ist.

2. System nach Anspruch 1, wobei das stellenunabhängige System ferner konfiguriert ist zum:
Empfangen, zu einem zweiten Zeitpunkt, einer zweiten Vielzahl von elektronischen Signalen von der Vielzahl von Elektroden;
Bestimmen, dass die zweite Vielzahl von elektronischen Signalen dem Gewebekontaktprofil entspricht; und
Bestimmen, dass der Katheter mit der Stelle in Kontakt ist, basierend auf dem Bestimmen, dass die zweite Vielzahl von elektronischen Signalen dem Gewebekontaktprofil entspricht.

3. System nach Anspruch 2, wobei das Bestimmen, dass die zweite Vielzahl von elektronischen Signalen dem Gewebekontaktprofil entspricht, das Bestimmen umfasst, dass ein Impedanzwert, der der zweiten Vielzahl von elektronischen Signalen entspricht, größer ist als ein Impedanzwert des Gewebekontaktprofils.

4. System nach Anspruch 1, wobei die Impedanzschwelle für die Stelle auf einer Impedanzänderung basiert.

5. System nach Anspruch 1, wobei die Impedanzschwelle für die Stelle auf einem Prozentsatz der Impedanzänderung basiert.

6. System nach Anspruch 1, wobei das Gewebekontaktprofil eine oder mehrere Impedanzschwellen für Elektroden des stellenunabhängigen Systems umfasst.

## Revendications

1. Système (20) permettant de déterminer un profil de contact avec les tissus afin de déterminer la proximité d'un cathéter avec les tissus, le système comprenant :
un cathéter (310) comprenant une pluralité d'électrodes (320) configurées pour détecter une première pluralité de signaux électroniques dans un organe intra-corporel ;
un système sensible à l'emplacement (340) configuré pour :
recevoir un sous-ensemble de signaux électroniques de la première pluralité de signaux électroniques ;
déterminer une valeur d'impédance sur la base du sous-ensemble de signaux électroniques ;
déterminer que la valeur d'impédance est supérieure à un seuil de valeur d'impédance de l'emplacement ;
déterminer l'emplacement du cathéter dans l'organe intra-corporel ; et
déterminer que le cathéter est en contact avec le tissu de l'organe intra-corporel à l'endroit indiqué, sur la base du sous-ensemble de signaux électroniques et d'au moins une propriété du tissu à l'endroit indiqué, dans lequel la propriété du tissu est un seuil d'impédance pour l'emplacement ;
un système agnostique d'emplacement (330) configuré pour :
recevoir la première pluralité de signaux électroniques provenant chacun de la pluralité d'électrodes respectives du cathéter ; et
générer, dans un premier temps, le profil de contact avec le tissu afin de déterminer la proximité du cathéter avec le tissu en fonction de la détermination, par le système de localisation, que le cathéter est en contact avec le tissu à l'emplacement considéré,
dans lequel le profil de contact avec le tissu comprend des valeurs d'impédance déterminées par le système agnostique d'emplacement pour la pluralité d'électrodes lorsque le système sensible à l'emplacement a déterminé que le cathéter est en contact avec le tissu à l'emplacement.

2. Système selon la revendication 1, dans lequel le système agnostique d'emplacement est configuré en outre pour :
recevoir, à un second moment, une seconde pluralité de signaux électroniques provenant de la pluralité d'électrodes ;
déterminer que la seconde pluralité de signaux électroniques répond au profil de contact avec le tissu ; et
déterminer que le cathéter est en contact avec l'emplacement sur la base de la détermination que la seconde pluralité de signaux électroniques répond au profil de contact avec le tissu.

3. Système selon la revendication 2, dans lequel la détermination que la seconde pluralité de signaux électroniques répond au profil de contact tissulaire comprend la détermination qu'une valeur d'impédance correspond à la seconde pluralité de signaux électroniques est supérieure à une valeur d'impédance du profil de contact tissulaire.

4. Système selon la revendication 1, dans lequel le seuil d'impédance pour l'emplacement est basé sur un changement d'impédance.

5. Système selon la revendication 1, dans lequel le seuil d'impédance pour l'emplacement est basé sur un pourcentage de changement d'impédance.

6. Système selon la revendication 1, dans lequel le profil de contact avec les tissus comprend un ou plusieurs seuils d'impédance pour les électrodes du système agnostique d'emplacement.
